# EUROPEAN PATENT SPECIFICATION

(11) **EP 3 534 880 B1**
(45) Date of publication and mention of the grant of the patent: **18.05.2022**
(21) Application number: 17817874.5
(22) Date of filing: 30.10.2017
(51) Int. Cl.: A61K 9/08, A61K 47/44, A61K 31/426, A61K 33/08, A61K 33/10, A61P 1/04

(54) **LIQUID ORAL PHARMACEUTICAL DOSAGE FORM COMPRISING AN HISTAMINE H2-RECEPTOR ANTAGONIST AND AN ANTACID**
FLÜSSIGE ORALE PHARMAZEUTISCHE ZUBEREITUNG ENTHALTEND EINEN HISTAMIN-H2-REZEPTOR-ANTAGONISTEN UND EIN ANTACIDUM
PRÉPARATION LIQUIDE ORALE PHARMACEUTIQUE COMPRENANT AN ANTAGONIST D'UN RECEPTEUR D'HISTAMIN H2 ET UN ANTI-ACIDE

(30) Priority: 01.11.2016 SE 1651441
(43) Date of publication of application: 11.09.2019
(73) Proprietor: Johnson & Johnson Consumer Inc., Skillman, NJ 08558 (US)
(72) Inventor: MUHAMMED, Salih Muhsin, Hyllinge 26574 (SE); LINDELL, Katarina, 25109 Helsingborg (SE); SIVERSSON, Carina, 25109 Helsingborg (SE)
(74) Representative: Carpmaels & Ransford LLP
(86) International application number: PCT/IB2017/056730
(87) International publication number: WO 2018/083583

(56) References cited:
- EP-A2- 2 085 079
- WO-A1-94/12180
- WO-A1-95/01780
- GB-A- 2 218 333
- US-A- 5 028 432
- US-A- 5 229 137
- US-A- 5 976 578

## Description

### FIELD OF INVENTION

The invention relates to a multiple compartment package containing liquid oral pharmaceutical dosage form, comprising a) pharmacologically effective amounts of at least one histamine H2- receptor antagonist in a hydrophobic/lipophilic liquid substantially free from water and b) pharmacologically effective amounts of at least one antacid, wherein a) and b) are physically separated from each other, as well as a method of treating a gastric disease or disorder by use of the liquid oral pharmaceutical dosage form.

### BACKGROUND OF INVENTION

Histamine H2 -receptor antagonists, for example cimetidine, ranitidine, nizetidine, roxatine and famotidine, reduce acid secretion by acting directly on the acid-secreting parietal cell located within the gastric gland of the stomach wall.

Although histamine H2 -receptor antagonists are remarkably effective in the treatment of many gastric disorders, in particular peptic and gastric ulcers, there exist certain patient groups which do not respond to treatment. In addition, the time lapse between dosing and onset of action, limits the potential benefit of histamine H2 -receptor antagonists in the treatment of acute, self-limiting gastric disorders.

Histamine H2 -receptor antagonists are of potential benefit in the self-medication of acute, self-limiting gastric disorders such as hyperacidity. However, their slow onset of action is unlikely to meet the consumer requirement for rapid relief of symptoms.

Co-administration of histamine H2 -receptor antagonists and other pharmaceutically active materials, including antacids, has been investigated. The rationale for co-administration with antacid is that the antacid brings about rapid relief from the symptoms of excess stomach acidity by neutralization whereas the histamine H2 -receptor antagonist acts independently by inhibiting secretion of acid from the parietal cell.

Antacids used today are made from a variety of inorganic salts such as calcium carbonate, sodium bicarbonate, magnesium salts and aluminum salts. Magnesium hydroxide and aluminum hydroxide are the most potent magnesium and aluminum salts and are often used in combination. In addition, magnesium oxide, magnesium carbonate, aluminum phosphate, magaldrate, magnesium trisilicate, and aluminum sucrose sulfate (sucralfate) are also employed.

So far it has been impossible to co-administrate histamine H2-receptor antagonists with antacids in a liquid form. It is well-known that histamine H2-receptor antagonist, i.e., famotidine is very unstable and thus difficult to produce a stable liquid formulation. Famotidine starts to degrade upon contact with water or any hydrolyzing agent as well as the antacids. So far there has been no product on the market comprising famotidine in a liquid form due to the stability problems. There have been a number of reports on the stability problems, when famotidine is dissolved in a liquid. However, antacids can be provided in the liquid form dissolved in a water based liquid without any stability problem.

US5229137, (priority date of 1992) discloses the use of antacids and famotidine. Both doses can be liquids. The document contains 5 examples. 4 relate to tablets and one example (2) relates to two liquid formulations. However, there is no indication in the application how a person skilled in the art should or would obtain the two liquid formulations from or how to prepare them in the laboratory. There is nothing, about how to prepare a histamine H2-receptor antagonist liquid formulation. In relation to the antacids it is only a reference to a Trade Mark, i.e., Maalox-Plus or Mylanta II and it is not possible for a person skilled in the art to find out what kind of formulation was behind a Trade Mark 1992. In addition, the product behind a Trade Mark changes over time and thus it is impossible for a person skilled in the art at the time when the invention was made to find out the product behind Maalox-Plus, 1992.

EP1992345 discloses a synergic combination comprising antacids, histamine H2-receptor antagonists and an anti-flatulence agent. It is mentioned that the composition may be formulated into a suspension. However, there is no guidance and no examples in the application how to formulate such a suspension and a person skilled in the art being aware of that famotidine in unstable would not find any guidance how to make such formulations.

EP2085079 discloses "a method to stabilize an alkaline pH for a H2 receptor inhibitor such as Famotidine through a microencapsulation process using as film forming materials, wax or polymer nature products, such as microcrystalline wax, Montan wax, ozokerite wax, chitosan or sodium alginate".

### SUMMARY OF THE INVENTION

The invention relates to the stability problem with histamine H2-receptor antagonists and how to stabilize those in a liquid formulation. However, so far the attempts to develop a ready to use stable liquid with a histamine H2-Receptor antagonist, such as famotidine have been unsuccessful, and such a liquid formulation is normally only stable for about a month in a refrigerator, not consumer friendly. Additionally, it is for the first time possible to produce a unit dose with at least the antacids and the histamine H2-receptor antagonist in a liquid form, but physically separated from each other. A liquid form is the preferred form for a significant fraction of the humans in need thereof.

In a first aspect, the invention relates to a multiple compartment package containing a liquid oral pharmaceutical dosage form, comprising a) pharmacologically effective amounts of at least one histamine H2 receptor antagonist in a hydrophobic/lipophilic liquid substantially free from water and b) pharmacologically effective amounts of at least one antacid in a liquid, wherein a) and b) are physically separated. A ready to use dosage form, consumer friendly, no need to be stored in a refrigerator as well as suitable for people on the go. A small discrete dosage form easy to ingest and which protect the histamine H2 receptor antagonist, such as famotidine from being degraded. The viscosity of the liquids in a) and b) might be about the same to secure that the histamine H2 receptor antagonist and the antacid(s) are properly consumed.

The invention relates to a package comprising multiple liquid oral pharmaceutical dosage forms.

In a second aspect, the invention relates to a method of treating a gastric disease or disorder by use of the liquid oral pharmaceutical dosage form comprising pharmacologically effective amounts of at least one histamine H2 receptor antagonist and pharmacologically effective amounts of at least one antacid as disclosed in the application.

### DETAILED DESCRIPTION OF EMBODIMENTS OF THE INVENTION

### Definitions

In the context of the present application and invention the following definitions apply:
The terms "physical barrier", "physically separated" are intended to mean that the histamine H2-receptor antagonist is separated from the antacid so that they have no contact at all during shelf life. The physical barrier prevents that the histamine H2-receptor antagonist gets in contact with any component that could change and or degrade the histamine H2-receptor antagonist during the shelf life.

The term "substantially free from water or free from water" is intended to mean that the content of water present in the composition is less than about 2 w% based on the total wt.% of the composition, such as less than 1.5,1, 0.5, 0.4, 0.3, 0.2 or less than 0.1 or totally free from water, i.e., 0 wt% based on the total wt.% of the composition.

The term "%w/w" is intended to mean the percentage of an ingredient(s)/ the total percentage by weight of the composition (100 %).

The term "histamine H2-receptor antagonist" is intended to mean an agent that inhibit histamine action and therefore reduce gastric secretion of the amount of acid produced and which is pharmacologically accepted.

The term "antacids" is intended to mean agents that function by neutralizing gastric acid and which is pharmacologically accepted.

The term "Medium Chain Triglyceride(s) (MCTs)" is/are intended to mean triglycerides whose fatty acids have an aliphatic tail of 6-12 carbon atoms.

The fatty acids found in MCTs are called medium-chain fatty acids (MCFAs). Like all triglycerides, MCTs are composed of a glycerol backbone and three fatty acids. In the case of MCTs, 2 or 3 of the fatty acid chains attached to glycerol are medium-chain in length. Examples includes, hexanoic acid (C6:0, common name caproic acid), octanoic acid (C8:0, common name caprylic acid), and decanoic acid (C10:0, common name capric acid) as well as dodecanoic acid (C12:0, common name lauric acid).

The term "pharmaceutically effective amount" includes an amount effective, at dosage and for periods of time necessary, to achieve the desired results. An effective amount of a compound may vary according to factors, such as intended posology, the disorder or disease state, age and weight of the subject.

The term "gastric disease or disorder" is primarily intended to mean an increased production of the acid secretion which leads to heartburn and bothersome gas symptoms in a subject also named indigestion. Indigestion, also known as dyspepsia, is a condition of impaired digestion. Symptoms may include upper abdominal fullness, heartburn, nausea, belching, or upper abdominal pain. People may also experience feeling full earlier than expected when eating. Dyspepsia is a common problem and is frequently caused by gastroesophageal reflux disease (GERD) or gastritis.

### Liquid oral pharmaceutical dosage form

The invention relates to a multiple compartment package containing a liquid oral pharmaceutical dosage form, comprising pharmacologically effective amounts of at least one histamine H2-receptor antagonist and pharmacologically effective amounts of at least one antacid, wherein there is a physical barrier in between the histamine H2-receptor antagonist and the antacid(s). The liquid dosage form is the first liquid dosage form in which a histamine H2-receptor antagonist is stable over time.

The at least one histamine H2-receptor antagonist and the at least one antacid are physically separated from each other, i.e., not get in contact prior to that the subject consumes the liquid oral pharmaceutical dosage form to protect the histamine H2-receptor antagonist from degradation.

The histamine H2-receptor antagonist is in a hydrophobic/lipophilic liquid being substantially free from water. The hydrophobic/lipophilic liquid may be an oil or a mixture thereof. Examples include medium chain triglycerides, olive oil, coconut oil, flaxseed oil, palm oil, palm kernel oil, ethyl oleate or a synthetic oil. The oil may also be a super refined oil such as castor oil, corn oil, cottonseed coil, peanut oil, safflower oil, sesame oil, medium chain triglycerides or soybean oil. By super refined oil is for example meant that the polar impurities present in triglycerides are usually comprised of monoglycerides, diglycerides, free fatty acids, plant sterols, coloring matter (chlorophyll, carotene) and oxidation products as well as other polar substances such as environmental chemicals are removed from the oil, which gives some new characteristics to the oil. Super refined oils may be obtained from Croda International Inc. (http://www.crodahealthcare.com). Simethicon or dimethicone or mixtures thereof may also be used either to replace or together with the hydrophobic/lipophilic liquid, as well as acting as an antifoaming agent to reduce bloating, discomfort and pain.

The hydrophobic/lipophilic liquid may further contain one or more excipients or pH regulating agents, such as sweeteners, flavors, cooling agents, preservatives or colorants. Examples of excipients that are useful are mentioned below. One of the purposes is to provide a dosage form which gives a smooth coating of the throat.

The H2 receptor antagonist is selected from the group consisting of cimetidine, ranitidine, nizatidine, roxatidine and famotidine, their pharmaceutically acceptable salts. One example is famotidine (see the examples below).

The one or more antacids may be selected from the group consisting of calcium carbonate, sodium bicarbonate, magnesium hydroxide, aluminum hydroxide, magnesium oxide, magnesium carbonate, aluminum phosphate, magaldrate, magnesium trisilicate, such as selected from the group consisting of calcium carbonate, sodium bicarbonate, magnesium hydroxide and aluminum hydroxide. One example is the combination of calcium carbonate and magnesium hydroxide or aluminum hydroxide and magnesium hydroxide.

The antacids may be in an aqueous-based liquid comprising one or more excipients and/or a pH adjusting agent. However, to get the same viscosity on the histamine H2 receptor antagonist and the antacid(s) liquids in the two separate compartments it is suitable to use one or more viscosity enhancing agents such as polysaccharides, cellulose, carboxymethylcellulose sodium and micorcrystalline cellulose or mixtures thereof as well as synthetic versions thereof in the antacid(s) liquid as well as the histamine H2 receptor antagonist liquid so that the viscosity of the two liquids are similar. Examples of such agents are gum such as xanthan gum and a mixture of carboxymethylcellulose sodium and micorcrystalline cellulose. The amount of xanthan gum is from about 0.05 to about 0.5 %w/w such as 0.1, 0.12, 0.2, 0.25, 0.3, 0.38, 0.4 %w/w and the amount of carboxymethylcellulose sodium and microcrystalline cellulose (Avicel CL 611^{®}) is from about 0.4 to about 2 %w/w, such as 0.5, 0.6, 0.62, 0.63, 0.7, 0.71, 0.8, 0.85, 0.9, 1.0, 1.1, 1.2, 1.25, 1.5, 1.6, 1.7, 1.8, 1.9 %w/w.

Alternatively, the antacids may be in a hydrophobic/lipophilic liquid being substantially free from water. The hydrophobic/lipophilic liquid may be an oil or a mixture thereof. Examples include medium chain triglycerides, olive oil, coconut oil, flaxseed oil, palm oil, palm kernel oil, ethyl oleate or a synthetic oil. The oil may also be a super refined oil such as castor oil, corn oil, cottonseed coil, peanut oil, safflower oil, sesame oil, medium chain triglycerides or soybean oil. By super refined oil is for example meant that the polar impurities present in triglycerides are usually comprised of monoglycerides, diglycerides, free fatty acids, plant sterols, coloring matter (chlorophyll, carotene) and oxidation products as well as other polar substances such as environmental chemicals are removed from the oil, which gives some new characteristics to the oil. Super refined oils may be obtained from Croda International Inc. (http://www.crodahealthcare.com). Famotidine may be formulated with other active ingredients like simethicone to control gas or alginic acid or salts thereof to act as a physical barrier.

Simethicon or dimethicone or mixtures thereof and other solvents/excipients may also be used either to replace or together with the hydrophobic/lipophilic liquid as well as acting as an antifoaming agent to reduce bloating, discomfort and pain in a hydrophobic/lipophilic liquid, such as those mentioned above related to the H2 receptor antagonist. Example of flavors suitable for the histamine H2 receptor antagonist liquid and/or the antacid(s) liquid includes peppermint, licorice, bubble gum, vanilla, caramel, red berries, such as strawberry, black current, blue berry and cherry, mint and lemon.

The liquid compositions of the invention are suspensions containing the active ingredients in admixture with pharmaceutically acceptable excipients typically found in suspensions for oral administration. Such excipients may be suitable suspending agents, for example, sodium alginate, polyvinylpyrrolidone, gum tragacanth, gum acacia, xanthan gum, locust bean gum and cellulose derivatives such as sodium carboxymethylcellulose, microcrystalline cellulose, hydroxy ethylcellulose, methyl cellulose or hydroxypropyl methylcellulose or mixtures thereof. Also included may be dispersing or wetting agents such as sorbitan esters or lecithin, antigelling additives, surface modifiers, aqueous or non-aqueous vehicles such as sorbitol solution, ethyl alcohol or fractionated vegetable oils, or diluents.

Sometimes if necessary a preservative component may be used. Such a preservative component may be selected from any pharmaceutically acceptable preservative. The alkyl esters of para-hydroxybenzoic acid (the parabens, e.g. butylparaben, methylparaben and propylparaben) are examples and may be used alone or in combination. Generally, the parabens are used in a concentration of about 0.02% w/w. Other preservatives include ethylenediamine tetra-acetic acid, propyl-p-hydroxybenzoates, antioxidants or sorbic acid. The compositions may also contain colorants and/or sweeteners as appropriate. The sweetening agents may be for example bulk sweeteners such as sugars (e.g. sucrose or fructose) or polyols (e.g. maltitol, xylitol, sorbitol, sucralose) and/or intense sweeteners such as saccharin, aspartame or acesulfame K.

Other active agents may be added to the preparation. For instance, antiflatulents, analgesics, antidiarrhea, antispasmodic agents or anti-foaming agents like simethicone may be added as well as other gastrointestinal agents in dosage amounts conventionally used in the treatment of gastrointestinal dysfunction, including indigestion.

Examples of liquid oral pharmaceutical dosage form include multiple compartment package, such as sachets or stick-packs, wherein one of the compartments comprises H2 receptor antagonist, such as famotidine, and the other compartment comprises one or more antacids. The compartments can be separated from each other by means of a perforation line, and be provided with an easy opening on one side.

In one embodiment the liquid oral pharmaceutical dosage form, comprising
a) pharmacologically effective amounts of famotidine in MCT substantially free from water and
b) pharmacologically effective amounts of calcium carbonate and magnesium hydroxide in a liquid and at least one flavor, wherein a) and b) are physically separated from each other and wherein a) and b) have similar viscosity and thus comprises viscosity enhancing agents.

### Dosage of the liquid oral pharmaceutical dosage form

The histamine H2-receptor antagonist such as famotidine may be present in an amount of from about 2 mg to about 30 mg, such as 4 mg to 20 mg or 8 mg to 12 mg or 2, 3, 4, 5, 6, 7, 8, 9, 10, 11, 12, 13, 14, 15, 16, 17, 18, 19, 20, 21, 22, 23, 24, 25, 26, 27, 28, 29 or 30 mg.

The antacid may be present in an amount of from about 200 to about 3 000 mg. If two different antacids are utilized they may be in the same amount or different amounts depending on the specific combinations. Examples are a liquid oral pharmaceutical dosage form having calcium carbonate in an amount from about 400 to about 1000 mg, such as 600, 700, 800, 900 or 1000 mg and magnesium hydroxide may be present in an amount from about 50 to about 300 mg, such as about 100- about 200 mg, such as 100, 110, 120, 130, 140, 150, 160, 165, 170, 180, 190 or 200 mg. If aluminum oxide is used it will be used in an amount from about 200 to about 600 mg, such as 300, 400, 416, 500 or 600 mg.

The liquid in respectively a) or b) may be in an amount of from about 2 to about 20 ml, such as about 2 to about 10 ml, such as 2, 3, 4, 5, 6, 7, 8, 9 or 10 ml. The amount in a) and b) may be the same or different depending on how a) and b) are formulated as well as depending on the form of the dosage form.

The invention relates to a package comprising multiple liquid oral pharmaceutical dosage forms as defined above.

Finally the invention relates to a method of treating a gastric disease or disorder by use of the liquid oral pharmaceutical dosage form as disclosed above.

In order to further illustrate the present invention and the advantages thereof, the following specific examples are given, it being understood that these examples are intended only to be illustrations without serving as a limitation on the scope of the present invention.

### EXAMPLES

### EXAMPLE 1

### MATERIALS

All oils were obtained from Croda International.

Magnesium hydroxide and Calcium carbonate were obtained from Magnesia Gmbh. Famotidine was obtained from Gedeon Richter in Hungary and film coated in-house using conventional coating technology, well known for a person skilled in the art.

### FORMULATIONS

Different kinds of formulations were produced and shown in Table 1.

Famotidine was mixed in different kinds of solvents including different oils to investigate which oils being suitable to be used together with famotidine (Sample 1-11 and 22-25 in Table 1). The results indicated that famotidine is stable in all oils but were most stable in the presence of Medium Chain Triglycerides (Sample 8F1/F2, 22C, 23A/B, 24 A and 25B7). Different kinds of antacid mixtures where investigated to identify which could be suitable (Sample 12-21 in Table 1).

### TWO COMPARTMENT PACKAGE

A two compartment package were produced being a double compartment sachet made from laminated aluminum foil. Three layers of aluminum foil were sealed to each other to create the two compartment package to be filled with the different formulations. The sachet was produced to allow opening of both compartments simultaneously. 5 ml of Sample 8 (uncoated famotidine) were introduced in one of the compartments and 5 ml of sample 12 were introduced into the other compartment. The two compartment package was sealed.

**Table 1**

| Sample | Sample composition | Amount |
|---|---|---|
| 1 A/B | FMT uncoated + Glycerol | 250 mg + 50ml / 500 mg + 50ml |
| 2 A/B | FMT uncoated + Glycerol | 125 mg + 25ml / 250 mg + 25ml |
| 3 A1/A2 | FMT uncoated/coated + Super refined Safflower oil | 25 mg + 5ml / 290 mg + 5ml |
| 4 B1/B2 | FMT uncoated/coated + Super refined Cottonseed oil | 25 mg + 5ml / 290 mg + 5ml |
| 5 C1/C2 | FMT uncoated/coated + Super refined Soybean oil | 25 mg + 5ml / 290 mg + 5ml |
| 6 D1/D2 | FMT uncoated/coated + Super refined Corn oil | 25 mg + 5ml / 290 mg + 5ml |
| 7 E1/E2 | FMT uncoated/coated + Super refined Sesame oil | 25 mg + 5ml / 290 mg + 5ml |
| 8 F1/F2 | FMT uncoated/coated + Super refined Medium chain triglycerides | 25 mg + 5ml / 290 mg + 5ml |
| 9 G1/G2 | FMT uncoated/coated + Super refined Ethyl Oleate | 25 mg + 5ml / 290 mg + 5ml |
| 10 H1/H2 | FMT uncoated/coated + Olive oil | 25 mg + 5ml / 290 mg + 5ml |
| 11 A | FMT uncoated + PEG+ Standard Medium chain triglycerides | |
| 12 A | Magnesium hydroxide heavy + Calcium carbonate heavy + water | 165 mg + 800 mg + 4035 mg |
| 13 A | Magnesium hydroxide heavy + Calcium carbonate heavy + Methocel HPMC E3 + water | 165 mg + 800 mg +500 mg + 3535 mg |
| 14 C | Magnesium hydroxide heavy + Calcium carbonate heavy + Methocel HPMC E3 + Sucralose + Acesulfame K + Flavor + Color + water | 165 mg + 800 mg +500 mg + 30 mg + 15 mg + 50 mg + 5mg + 3535 mg |
| 15 D | Magnesium hydroxide heavy + Calcium carbonate heavy + Methocel HPMC E3 + water | 165 mg + 800 mg +1000 mg + 3535 mg |
| 16 A1 | Magnesium hydroxide heavy + Calcium carbonate heavy + Super refined Ethyl Oleate | 165 mg + 800 mg + 5 ml |
| 17 A2 | Magnesium hydroxide heavy + Calcium carbonate heavy + Super refined Medium chain triglycerides | 165 mg + 800 mg + 5 ml |
| 18 B | Magnesium hydroxide heavy + Calcium carbonate heavy + water | 165 mg + 800 mg + 5 ml |
| 19 E/C | FMT uncoated + Simethicone | 10 mg + 2 ml / 20 mg + 4 g |
| 20 A | Magnesium hydroxide + Calcium carbonate light + Super refined Medium chain triglycerides | 165 mg + 800 mg + 5 ml |
| 21 B | Magnesium hydroxide + Calcium carbonate light + Water | 165 mg + 800 mg + 5 ml |
| 22 C | FMT uncoated + Super refined Medium chain triglycerides | 10 mg + 5 ml |
| 23 A/B | FMT uncoated + Super refined Medium chain triglycerides | 5 mg + 20 ml / 5 mg + 10 ml |
| 24 A | FMT uncoated + Simethicone + Super refined Medium chain triglycerides | 100 mg + 5000 mg + 24900 mg |
| 25 B7 | FMT uncoated + Standard Medium chain triglycerides | 80 mg + 39 920 mg |

### EXAMPLE 2

Analysis on the stability of famotidine suspended in different oils.

Uncoated famotidine was mixed in one of the following oils Safflower oil (B1), Soybean oil (B2), Sesame oil (B3), Corn oil (B4), Cottonseed oil (B5), Super refined MCT (B6) and standard MCT (B7). 3 samples were prepared for each batch and the samples where incubated at 40/75 °C, 50 °C or 60 °C. 10 mg famotidine was mixed with 5 g oil. Samples were removed after 14 days, 1 month, 2 month and 3 month and the stability of famotidine evaluated.

For the stability analysis, the samples were prepared and analyzed using the method below

### Solutions

| **Buffer (50 mM Sodium/Potassium Phospate, pH 6.2)** | |
|---|---|
| di-Sodiumhydrogen phosphate (Na₂HPO₄) dehydrate (g) | 1.64 |
| Potassium phosphate monobasic (K₃PO₄) (g) | 5.55 |
| Milli-Q up to (ml) | 1000 |

| Diluent | |
|---|---|
| Methanol (ml) | 200 |
| Buffer up to (ml) | 1000 |

### Mobile Phases

| **Mobile Phase A** | |
|---|---|
| Buffer (ml) | 200 |
| Milli-Q (ml) | 780 |
| Acetonitrile (ml) | 20 |
| Potassium hexafluorophospate (KPF₆) (g) | 7.36 |
| Mix and filtrate (nylon 0.45 µm filter) | |

| **Mobile phase B** | |
|---|---|
| Buffer (ml) | 200 |
| Milli-Q (ml) | 100 |
| Acetonitrile (ml) | 700 |
| Potassium hexafluorophospate (KPF₆) (g) | 7.36 |
| Mix and filtrate (nylon 0.45 µm filter) | |

### Famotidine standards

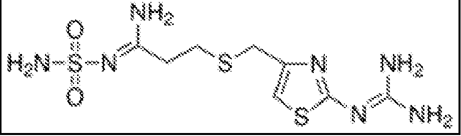

Famotidine: CAS nr: 76824-35-6, C₈H₁₅N₇O₂S₃, MW: 337.45

| **Fam Stock (400 µg FAM /ml)** | |
|---|---|
| Famotidine (mg) in 200ml volumetric flask | 80 |
| Metanol (ml) | Ca 125 |
| Dissolve with ultrasound bath | |
| Methanol upp till (ml) | 200 |

| **Fam Std (80 µg/ml)** | |
|---|---|
| Fam Stock (ml) | 20 |
| Buffert up to (ml) | 100 |

| **Fam Std (40 µg/ml)** | |
|---|---|
| Fam std (80 µg/ml) | 10 |
| Diluent up to (ml) | 20 |

| **Fam Std (8 µg/ml)** | |
|---|---|
| Fam std (80 µg/ml) (ml) | 1 |
| Diluent up to (ml) | 10 |

| **SST** | |
|---|---|
| SST (Ph.Eur Fam SST) | 1 speck |
| Diluent up to (ml) | 1 |

### Sample preparation

- Pour sample of sample bottle into a 100 ml volumetric flask.
- Rinse the vial at least 3 times with diluent to the volumetric flask.
- Fill the volumetric flask to about 70 ml with diluent.
- Shake the sample for 45 minutes.
- Fill the volumetric flask up to 100 ml with diluent.
- Mix the sample.
- Filter the sample and transfer to LC-vial for LC-UV analysis. (MilleX HV Hydrophilic PVDF 0.45 µm)

### Instrument parameters

| Column: | ACE, C8, 3µm, 150mmx4.6mm | | |
|---|---|---|---|
| Flow: | 1.0ml/min | | |
| Gradient: | | % MobfasA | %MobfasB |
| Time(min) | | | |
| | 0.0 | 100 | 0 |
| | 1.0 | 100 | 0 |
| | 16.0 | 35 | 65 |
| | 16.1 | 100 | 0 |
| | 18.0 | 100 | 0 |
| Injection volume: | 10 µl | | |
| Column temp: | 35°C | | |
| Detection: | UV, 278 nm | | |

| **Assay** | | | | | | |
|---|---|---|---|---|---|---|
| **Sample No** | **Storage Cond** | **0** | **14 days** | **1 month** | **2 months** | **3 months** |
| B1 | 40/75 | 93% | 98% | 95% | 84% | 90% |
| B1 | 50°C | 93% | 99% | 91% | 88% | na |
| B1 | 60°C | 93% | 96% | 94% | 82% | na |
| B2 | 40/75 | 98% | 88% | 81% | 81% | 85% |
| B2 | 50°C | 98% | 102% | 92% | 81% | na |
| B2 | 60°C | 98% | 93% | 93% | 72% | na |
| B3 | 40/75 | 96% | 84% | 97% | 88% | 87% |
| B3 | 50°C | 96% | 101% | 95% | 84% | na |
| B3 | 60°C | 96% | 98% | 88% | 68% | na |
| B4 | 40/75 | 91% | 88% | 90% | 77% | 84% |
| B4 | 50°C | 91% | 90% | 84% | 80% | na |
| B4 | 60°C | 91% | 78% | 87% | 74% | na |
| B5 | 40/75 | 101% | 96% | 93% | 84% | 80% |
| B5 | 50°C | 101% | 100% | 91% | 73% | na |
| B5 | 60°C | 101% | 99% | 93% | 81% | na |
| B6 | 40/75 | 103% | 104% | 98% | 93% | 99% |
| B6 | 50°C | 103% | 99% | 97% | 88% | na |
| B6 | 60°C | 103% | 98% | 95% | 85% | na |
| B7 | 40/75 | 103% | 104% | 101% | 100% | 103% |
| B7 | 50°C | 103% | 103% | 101% | 96% | na |
| B7 | 60°C | 103% | 101% | 99% | 92% | na |

| | | | | | | |
|---|---|---|---|---|---|---|
| na means not analysed | | | | | | |

The results from the stability test showed that famotidine in stable in all oils but most stable when mixed with a MCT oil either standard purity or refined MCT (sample B6 and B7).

### EXAMPLE 3

Evaluation of different antacid formulation for their viscosity and consistency. Different amounts of Xanthan Gum 180 and Avicel CL 611 NF were used.

| Sample | | TE007 | TF0035 | TF0036 | TF0042 | TF0043 | TF0053 | TF0054 | TF0055 | TF0056 | TF0057 |
|---|---|---|---|---|---|---|---|---|---|---|---|
| Ingredients | Purpose | %ww | %ww | %ww | %ww | %ww | %ww | %ww | %ww | %ww | %ww |
| Calcium carbonate, CalEssence 70, | Active | 10,67 | 14,34 | 25,72 | 14,18 | 14,29 | 14,29 | 14,25 | 14,27 | 14,33 | 14,36 |
| Magnesium hydroxide, Magnesia 725 | Active | 2,21 | 2,96 | 5,31 | 2,93 | 2,95 | 2,95 | 2,94 | 2,94 | 2,96 | 2,96 |
| Sorbitol | Sweetener | 10,17 | 9,12 | 8,18 | 9,01 | 9,08 | 9,08 | 9,06 | 9,07 | 9,11 | 9,13 |
| Xylitol crystalline | Sweetener | 0,55 | 0,49 | 0,44 | 0,49 | 0,49 | 0,49 | 0,49 | 0,49 | 0,49 | 0,49 |
| Xanthan Gum 180 | Suspending agent | 0,14 | 0,13 | 0,11 | 0,12 | 0,13 | 0,13 | 0,12 | 0,13 | 0,25 | 0,38 |
| Avicel CL 611 NF | Suspending agent | 0,70 | 0,63 | 0,56 | 0,62 | 0,63 | 0,71 | 0,80 | 1,25 | 0,63 | 0,63 |
| Acesulfame K | Sweetener | 0,01 | | | | | | | | | |
| Sucralose | Sweetener | 0,01 | | | | | | | | | |
| Purified water | Solvent | 75,53 | 72,34 | 59,68 | 72,65 | 72,44 | 72,35 | 72,34 | 71,85 | 72,23 | 72,05 |
| Total volume (ml) | | 7500 | 5000 | 2500 | 5000 | 5000 | 5000 | 5000 | 5000 | 5000 | 5000 |

## Claims

1. A multiple compartment package containing a liquid oral pharmaceutical dosage form, wherein the liquid oral pharmaceutical dosage form comprises:
a) pharmacologically effective amounts of at least one histamine H2- receptor antagonist in a hydrophobic/lipophilic liquid substantially free from water; and
b) pharmacologically effective amounts of at least one antacid in a liquid,
wherein a) and b) are physically separated from each other in separate compartments of the multiple compartment package,
wherein, the hydrophobic/lipophilic liquid is an oil, wherein the oil is medium chained triglycerides.

2. The multiple compartment package according to claim 1, wherein the at least one H2-receptor antagonist is selected from the group consisting of cimetidine, ranitidine, nizatidine, roxatidine and famotidine, and pharmaceutically acceptable salts thereof, optionally wherein the at least one H2- receptor antagonist is famotidine.

3. The multiple compartment package according to claim 1 or claim 2, wherein the oil is super refined.

4. The multiple compartment package according to any of preceding claims, wherein the at least one antacid is selected from the group consisting of calcium carbonate, sodium bicarbonate, magnesium hydroxide, aluminum hydroxide, magnesium oxide, magnesium carbonate, aluminum phosphate, magaldrate and magnesium trisilicate.

5. The multiple compartment package according to claim 4, wherein the at least one antacid is selected from the group consisting of calcium carbonate, magnesium hydroxide and aluminum hydroxide.

6. The multiple compartment package according to claim 5, wherein the at least one antacid is selected from the group consisting of a combination of calcium carbonate and magnesium hydroxide or a combination of aluminum hydroxide and magnesium hydroxide.

7. The multiple compartment package according to any of preceding claims, wherein a) and/or b) comprises at least one flavor.

8. The multiple compartment package according to any of preceding claims, comprising simethicone or dimethicone or mixture thereof in a) and/or b).

9. The multiple compartment package according to claims 1-8, wherein a) and b) comprises a viscosity enhancing agent.

10. The multiple compartment package according to claim 9, wherein the viscosity enhancing agent is selected from the group consisting of gums, polysaccharides, cellulose, carboxymethylcellulose sodium and microcrystalline cellulose or mixtures thereof as well as synthetic versions thereof.

11. The multiple compartment package according to any of preceding claims, wherein the at least one H2- receptor antagonist is famotidine present in an amount of from about 2 to about 30 mg.

12. The multiple compartment package according to any of preceding claims, wherein the at least one antacid is present in an amount of from about 200 to about 3000 mg.

13. The multiple compartment package according to any of preceding claims, wherein the liquid formulations in each of a) and b) is present in an amount of from about 2 to about 20 ml.

14. A liquid oral pharmaceutical dosage form comprising:
a) pharmacologically effective amounts of at least one histamine H2- receptor antagonist in a hydrophobic/lipophilic liquid substantially free from water; and
b) pharmacologically effective amounts of at least one antacid in a liquid,
wherein, the hydrophobic/lipophilic liquid is an oil, wherein the oil is medium chained triglycerides, for use in a method of treating a gastric disease or disorder, wherein the method comprises providing the liquid oral dosage form in a multiple compartment package according to any of claims 1-13.

## Patentansprüche

1. Packung mit mehreren Teilbereichen, die eine flüssige orale pharmazeutische Darreichungsform enthält, wobei die flüssige orale pharmazeutische Darreichungsform Folgendes umfasst:
a) pharmakologisch wirksame Mengen an mindestens einem Histamin-H2-Rezeptor-Antagonisten, in einer hydrophoben/lipophilen Flüssigkeit, die im Wesentlichen wasserfrei ist; und
b) pharmakologisch wirksame Mengen an mindestens einem Antazidum in einer Flüssigkeit,
wobei a) und b) physisch voneinander getrennt in gesonderten Teilbereichen der Packung mit mehreren Teilbereichen vorliegen,
wobei es sich bei der hydrophoben/lipophilen Flüssigkeit um ein Öl handelt, wobei es sich bei dem Öl um mittelkettige Triglyceride handelt.

2. Packung mit mehreren Teilbereichen gemäß Anspruch 1, wobei der mindestens eine H2-Rezeptor-Antagonist aus der Gruppe ausgewählt ist, die aus Cimetidin, Ranitidin, Nizatidin, Roxatidin und Famotidin sowie deren pharmazeutisch unbedenklichen Salzen ausgewählt ist, wobei es sich bei dem mindestens einen H2-Rezeptor-Antagonisten möglicherweise um Famotidin handelt.

3. Packung mit mehreren Teilbereichen gemäß Anspruch 1 oder Anspruch 2, wobei das Öl hochraffiniert ist.

4. Packung mit mehreren Teilbereichen gemäß einem beliebigen der vorhergehenden Ansprüche, wobei das mindestens eine Antazid aus der Gruppe ausgewählt ist, welche aus Calciumcarbonat, Natriumhydrogencarbonat, Magnesiumhydroxid, Aluminiumhydroxid, Magnesiumoxid, Magnesiumcarbonat, Aluminiumphosphat, Magaldrat und Magnesiumtrisilicat besteht.

5. Packung mit mehreren Teilbereichen gemäß Anspruch 4, wobei das mindestens eine Antazid aus der Gruppe ausgewählt ist, welche aus Calciumcarbonat, Magnesiumhydroxid und Aluminiumhydroxid besteht.

6. Packung mit mehreren Teilbereichen gemäß Anspruch 5, wobei das mindestens eine Antazid aus der Gruppe ausgewählt ist, welche aus einer Kombination von Calciumcarbonat und Magnesiumhydroxid oder einer Kombination von Aluminiumhydroxid und Magnesiumhydroxid besteht.

7. Packung mit mehreren Teilbereichen gemäß einem beliebigen der vorhergehenden Ansprüche, wobei a) und/oder b) mindestens einen Geschmacksstoff umfasst.

8. Packung mit mehreren Teilbereichen gemäß einem beliebigen der vorhergehenden Ansprüche, wobei in a) und/oder b) Simethicon oder Dimethicon oder deren Mischung enthalten ist.

9. Packung mit mehreren Teilbereichen gemäß den Ansprüchen 1 bis 8, wobei a) und b) ein viskositätsförderndes Mittel umfassen.

10. Packung mit mehreren Teilbereichen gemäß Anspruch 9, wobei das viskositätsfördernde Mittel aus der Gruppe ausgewählt ist, welche aus Gummen, Polysacchariden, Cellulose, Carboxymethylcellulose-Natrium und mikrokristalliner Cellulose oder deren Mischungen sowie deren synthetischen Versionen besteht.

11. Packung mit mehreren Teilbereichen gemäß einem beliebigen der vorhergehenden Ansprüche, wobei es sich bei dem mindestens einen H2-Rezeptor-Antagonisten um Famotidin handelt, welches in einer Menge von ungefähr 2 bis ungefähr 30 mg vorliegt.

12. Packung mit mehreren Teilbereichen gemäß einem beliebigen der vorhergehenden Ansprüche, wobei das mindestens eine Antazidum in einer Menge von ungefähr 200 bis ungefähr 3000 mg vorliegt.

13. Packung mit mehreren Teilbereichen gemäß einem beliebigen der vorhergehenden Ansprüche, wobei die flüssigen Formulierungen in a) und b) jeweils in einer Menge von ungefähr 2 bis ungefähr 20 ml vorliegt.

14. Flüssige orale pharmazeutische Darreichungsform, die Folgendes umfasst:
a) pharmakologisch wirksame Mengen an mindestens einem Histamin-H2-Rezeptor-Antagonisten, in einer hydrophoben/lipophilen Flüssigkeit, die im Wesentlichen wasserfrei ist; und
b) pharmakologisch wirksame Mengen an mindestens einem Antazidum in einer Flüssigkeit,
wobei es sich bei der hydrophoben/lipophilen Flüssigkeit um ein Öl handelt, wobei es sich bei dem Öl um mittelkettige Triglyceride handelt, zur Verwendung in einem Verfahren zur Behandlung einer Erkrankung oder Störung des Magens, wobei im Rahmen des Verfahrens die flüssige orale Darreichungsform in einer Packung mit mehreren Teilbereichen gemäß einem beliebigen der Ansprüche 1 bis 13 bereitgestellt wird.

## Revendications

1. Conditionnement à compartiments multiples contenant une forme de dosage pharmaceutique orale liquide, dans lequel la forme de dosage pharmaceutique orale liquide comprend :
a) des quantités efficaces sur le plan pharmacologique d'au moins un antagoniste des récepteurs H2 de l'histamine dans un liquide hydrophobe/lipophile sensiblement exempt d'eau ;
et
b) des quantités efficaces sur le plan pharmacologique d'au moins un antiacide dans un liquide ;
où a) et b) sont séparés physiquement l'un de l'autre dans des compartiments séparés du conditionnement à compartiments multiples ;
où le liquide hydrophobe/lipophile est une huile, où l'huile est constituée de triglycérides à chaîne moyenne.

2. Conditionnement à compartiments multiples selon la revendication 1, dans lequel le au moins un antagoniste des récepteurs H2 est choisi dans le groupe constitué par la cimétidine, la ranitidine, la nizatidine, la roxatidine et la famotidine, et les sels pharmaceutiquement acceptables de celles-ci, éventuellement où le au moins un antagoniste des récepteurs H2 est la famotidine.

3. Conditionnement à compartiments multiples selon la revendication 1 ou la revendication 2, dans lequel l'huile est surraffinée.

4. Conditionnement à compartiments multiples selon l'une quelconque des revendications précédentes, dans lequel le au moins un antiacide est choisi dans le groupe constitué par le carbonate de calcium, le bicarbonate de sodium, l'hydroxyde de magnésium, l'hydroxyde d'aluminium, l'oxyde de magnésium, le carbonate de magnésium, le phosphate d'aluminium, le magaldrate et le trisilicate de magnésium.

5. Conditionnement à compartiments multiples selon la revendication 4, dans lequel le au moins un antiacide est choisi dans le groupe constitué par le carbonate de calcium, l'hydroxyde de magnésium et l'hydroxyde d'aluminium.

6. Conditionnement à compartiments multiples selon la revendication 5, dans lequel le au moins un antiacide est choisi dans le groupe constitué par une combinaison de carbonate de calcium et d'hydroxyde de magnésium ou une combinaison d'hydroxyde d'aluminium et d'hydroxyde de magnésium.

7. Conditionnement à compartiments multiples selon l'une quelconque des revendications précédentes, dans lequel a) et/ou b) comprennent au moins un arôme.

8. Conditionnement à compartiments multiples selon l'une quelconque des revendications précédentes, comprenant de la siméthicone ou de la diméthicone ou un mélange de celles-ci dans a) et/ou b).

9. Conditionnement à compartiments multiples selon les revendications 1-8, dans lequel a) et b) comprennent un agent d'amélioration de la viscosité.

10. Conditionnement à compartiments multiples selon la revendication 9, dans lequel l'agent d'amélioration de la viscosité est choisi dans le groupe constitué par les gommes, les polysaccharides, la cellulose, la carboxyméthylcellulose de sodium et la cellulose microcristalline ou des mélanges de ceux-ci, ainsi que des versions synthétiques de ceux-ci.

11. Conditionnement à compartiments multiples selon l'une quelconque des revendications précédentes, dans lequel le au moins un antagoniste des récepteurs H2 est constitué de famotidine présente selon une quantité allant d'environ 2 à environ 30 mg.

12. Conditionnement à compartiments multiples selon l'une quelconque des revendications précédentes, dans lequel le au moins un antiacide est présent selon une quantité allant d'environ 200 à environ 3000 mg.

13. Conditionnement à compartiments multiples selon l'une quelconque des revendications précédentes, dans lequel les formulations liquides dans chacun parmi a) et b) sont présentes selon une quantité allant d'environ 2 à environ 20 ml.

14. Forme de dosage pharmaceutique orale liquide, comprenant :
a) des quantités efficaces sur le plan pharmacologique d'au moins un antagoniste des récepteurs H2 de l'histamine dans un liquide hydrophobe/lipophile sensiblement exempt d'eau ;
et
b) des quantités efficaces sur le plan pharmacologique d'au moins un antiacide dans un liquide ;
où le liquide hydrophobe/lipophile est une huile, où l'huile est constituée de triglycérides à chaîne moyenne, pour une utilisation dans une méthode destinée au traitement d'une maladie ou d'un trouble gastrique, la méthode comprenant la mise à disposition de la forme de dosage orale liquide dans un conditionnement à compartiments multiples selon l'une quelconque des revendications 1-13.
